Europäisches Patentamt

European Patent Office

Office européen des brevets

(19)

(11) EP 1 728 516 A1

(12) **EUROPEAN PATENT APPLICATION**

(43) Date of publication:
06.12.2006 Bulletin 2006/49

(51) Int Cl.:
*A61K 38/17* (2006.01)     *A61K 47/48* (2006.01)
*C07K 17/00* (2006.01)     *A61P 9/04* (2006.01)

(21) Application number: 06116525.4

(22) Date of filing: 02.11.1999

(84) Designated Contracting States:
AT BE CH CY DE DK ES FI FR GB GR IE IT LI LU
MC NL PT SE

(30) Priority: 02.11.1998  US 106718 P
27.07.1999  US 145883 P

(62) Document number(s) of the earlier application(s) in
accordance with Art. 76 EPC:
99960187.5 / 1 126 866

(71) Applicant: **The Regents of the University
of California**
La Jolla, CA 92093-0910 (US)

(72) Inventors:
• **Chen, Kenneth**
La Jolla, CA 92093-0613-C (US)
• **Dillman, Wolfgang**
La Jolla, CA 92093-0613-C (US)
• **Minamisawa, Susanne**
La Jolla, CA 92093-0613-C (US)

• **He, Huaping**
La Jolla, CA 92093-0613-C (US)
• **Hoshijima, Masahiko**
La Jolla, CA 92093-0613-C (US)
• **Meyer, Markus**
La Jolla, CA 92093-0613 (US)
• **Scott, Christopher**
La Jolla, CA 92093-0613-C (US)
• **Wang, Yibin**
La Jolla, CA 92093-0613-C (US)
• **Silverman, Gregg J.**
La Jolla, CA 92093-0613-C (US)

(74) Representative: **Gates, Marie Christina Esther et al**
**Tomkins & Co.**
**5 Dartmouth Road**
**Dublin 6 (IE)**

Remarks:
This application was filed on 03 - 07 - 2006 as a
divisional application to the application mentioned
under INID code 62.

(54)    **A method for inhibition of phospholamban activity for the treatment of cardiac disease and
heart failure**

(57)    The present invention provides a method for the
treatment of heart failure through the use of small peptide
complexes and recombinant proteins which function to
enhance contractility in failing hearts and reduce blood
pressure in individuals with hypertension by inhibiting the
interaction between phospholamban and sarcoplasmic
reticulum $Ca^{2+}$ ATPase (SERCA2a) within cardiomyo-
cytes. IN addition, a means is provided for the transport
of such therapeutic agents into the cytoplasm and nucle-
us of cardiomyocytes.

Fig. 1

EP 1 728 516 A1

Printed by Jouve, 75001 PARIS (FR)

## Description

This application claims the benefit of priority of United States Provisional Application Serial No. 60/106,718, filed November 2, 1998 and United States Provisional Application Serial No. 60/145,883, filed July 27, 1999, both of which are incorporated herein by reference in their entirety.

## BACKGROUND OF THE INVENTION

### FIELD OF THE INVENTION

This invention relates generally to a method for the treatment of heart failure, and more specifically to the inhibition of phospholamban (PLB) activity for the treatment of myocardial dysfunction.

### BACKGROUND INFORMATION

Heart failure is the leading cause of combined morbidity and mortality in the United States and other developed countries. Congestive heart failure is characterized by a reduced contraction and delayed relaxation of the heart however, fundamental molecular mechanisms which drive the patho-physiological pathways for congestive heart are largely unknown. Current therapy for the heart disease is primarily palliative and is not targeted to the underlying biological pathways which are thought to lead to the initiation and progression of cardiac muscle dysfunction.

Heart muscle failure is a complex, integrative, multi-factorial disease in which the genetic pathways that confer susceptibility are interwoven with the environmental stimulus of biomechanical stress that accompanies heart injury, pressure and volume overload, and genetic defects in components of the cytoskeleton. In response to this biomechanical stress, a series of parallel and converging signaling pathways are activated that lead to the adaptive response of compensatory hypertrophy. Subsequently, there can be a transition to chamber dilation and pump failure that is associated with a loss of viable myocytes, a decrease in contractile elements, myofilament disarray and interstitial fibrosis.

Recently, the activation of signal transduction pathways which trigger the onset of programmed cell death have been implicated in promoting the pathological transition to heart failure, as well as a gp130 dependent myocyte survival pathway that can block the actions of the pro-apoptotic pathways and prevent the early onset of heart failure and cardiomyopathy. In addition to these extrinsic stress-related pathways for myocyte adaptation, there also must be intrinsic signaling pathways that lead to the impairment of cardiac excitation-contraction (EC) coupling and associated severe defects in cardiac contractile performance that are the clinical hallmarks of the progression of the heart failure phenotype.

The sarcoplasmic reticulum (SR) plays an integral role in the coordination of the movement of cytostolic $Ca^{2+}$ throughout the cardiac tissue. In separate studies by Mercadier, et al. (J. Clin. Invest.,1990; 85:305-309), Arai, et al. (Circ. Res., 1993; 72:463-469), de la Bastie, et al. (Circ. Res., 1990; 66:554-564), and Feldman, et al. (Circ. Res., 1993; 73:184-192), research on human failing hearts and animal models of heart failure have suggested that the reduced uptake the cytostolic $Ca^{2+}$ by the SR is responsible for prolonged diastolic relaxation. $Ca^{2+}$ stored in the SR is released into the cytosol to activate the contraction of cardiac muscle and subsequently re-accumulated to achieve relaxation. Activity of the cardiac SR $Ca^{2+}$ ATPase (SERCA2a) is the rate determining factor of $Ca^{2+}$ re-uptake into the SR, and SERCA2a activity is itself regulated by phospholamban, a 52-amino acid muscle-specific SR phosphoprotein.

Phospholamban (PLB) was first identified as a major phosphorylation target in the SR membrane in research by Tada, et al. (J. Bio.l Chem., 1974; 249:6174-6180) and appeared to be a potent inhibitor of SERCA2a activity in its unphosphorylated form. The inhibitory effect of PLB on SERCA2a is reduced by an increase in intracellular calcium or by the phosphorylation of PLB in response to β-adrenergic stimulation. PLB exists primarily in a pentameric form, that when subjected to high temperature, dissociates into five equivalent monomers.

The amino acids of monomeric PLB are grouped into three physical and functional domains. Domains Ia and II are rich in α-helices and are connected by the less structured domain Ib. Domain Ia is composed of amino acids 1-20, the majority of which are in an α-helical confirmation, having a net positive charge. Domain Ib consists of amino acid residues 21-30 and constitutes the cytoplasmic sector of the monomer. Domain II amino acids 31-52, represents the transmembrane sector and is made up solely of uncharged residues that are responsible for stabilizing the pentamer formation.

PLB is a mediator in the regulation of myocardial function by catecholamines through the cAMP cascade. Ser (16) and Thr (17), in domain Ia are the confirmed binding sites for cAMP-dependent protein kinase (PKA) and Ca/ calmodulin-dependent protein kinase, respectively, which function to catalyze phosphoester phosphorylation of PLB which in turn relieves its inhibition on SERCA2a activity. Because Ser (16) and Thr (17) can be phosphorylated by the kinases, the net charge of the amino acids can be shifted from positive to neutral and even to negative. Together with the charged residues of SERCA2a, the shifting of charges in domain Ia of PLB can result in profound alterations in the protein-protein interaction of the PLB-SERCA2a system. Domain II also contains some key amino acids for functional expression of PLB, in that amino acids of one face of the domain II helix are associated with the transmembrane domain of SERCA2a.

There may be two ways in which PLB regulates $Ca^{2+}$-ATPase activity: 1) a quick-acting, short-term mechanism involving PLB phosphorylation and depression of calcium pumping activity, and 2) a slower acting but longer term process involving a change in the molecular ratio of PLB to the $Ca^{2+}$-ATPase brought about by control of gene expression. Under physiological conditions, phosphorylation at Ser (16) by PKA is the predominant event that leads to proportional increases in the rate of $Ca^{2+}$ uptake to the SR and accelerates ventricular relaxation. An increase in the relative ratio of PLB to SERCA2a is an important determinant of SR dysfunction in both experimental and human heart failure. Moreover, attenuated PLB phosphorylation by PKA may be responsible for impaired diastolic function and prolonged $Ca^{2+}$ transients in failing hearts by which the β-adrenergic receptor-cAMP system is severely down-regulated by enhanced sympathetic tone.

A detailed mutagenesis study by Toyofuku, et al. (J. Biol. Chem., 1994; 269:3088-3094) revealed that several amino acids in the cytoplasmic domain of PLB are important for its inhibitory function. The study showed that when the certain amino acids were mutated into amino acids of different charge, the PLB mutants lost their inhibitory effect on the co-transfected SERCA2 in HEK293 cells. However, it is still unclear whether PLB bearing these mutants can exert dominant negative effects on endogenous wild-type PLB and consequently stimulate endogenous SERCA2a in cardiac myocytes. Additionally, it is unclear how the mechanisms of transfer of these PLB point mutations into cardiomyocytes breach the cytoplasmic membrane barrier in order to effect endogenous SERCA2a activity.

Genetically based mouse models of dilated cardiomyopathy by Arber, et al. (Cell, 88:393-403; 1997) provide evidence that chamber dilation and the progression to heart failure is dependent on a specific $Ca^{2+}$ cycling defect in the cardiac sarcoplasmic reticulum. In the mouse models, ablation of phospholamban (PLB) rescued the spectrum of structural, functional, and molecular phenotypes that resemble heart failure. Furthermore, release of the phospholamban-SERCA2a interaction through the forced *in vivo* overexpression of a PLB point mutation dominantly activated the contractility of ventricular muscle cells. Thus, there is the possibility that interfering with the PLB-SERCA2a interaction may provide a novel therapeutic approach for preventing heart failure.

There is the understanding that interfering with the PLB-SERCA2a interaction may be a potential therapeutic target for the treatment of heart failure, however, the internalization of exogenous molecules to enhance cardiac contractility by live myocytes remains an unsolved issue. A means must be available to deliver any therapeutic agent directly to the cytoplasm and nucleus of cardiac myocytes. Penetratins, a class of peptides with translocating properties, have the ability to carry hydrophillic compounds across the plasma membrane. Research by Schwarze, et al. (Science 285: 1569-1572; 1999) has demonstrated an approach to protein transduction using a penetratin-based fusion protein which contains an $NH_2$-terminal 11-amino acid protein transduction domain from the denatured HIV TAT protein (Genebank Accession No. AF033819) . Using this non-cell-type specific transfer system allows direct targeting of oligopeptides and oligonucleotides to the cytoplasm and nucleus. One of the most well characterized translocation peptides is one that corresponds to residues 43 to 58 of antennapedia, a Drosophila transcription factor. It is believed that the translocation peptide interacts with charged phospholipids on the outer side of the cell membrane. Destabilization of the bilayer results in the formation of inverted micelles containing the peptide that travels across the cell membrane and eventually open on the cytoplasmic side. While the use of transport peptides to move cargo molecules into cells is not novel, it has not been demonstrated that transport peptides work well in cardiomyocytes.

Thus the need remains for methods for the inhibition of PLB through the use of mutants or small molecule inhibitors of PLB in order to manipulate the PLB/SERCA2a interaction in cardiac myocytes, as well as a transport means for these mutants or small molecule inhibitors of PLB to cross sarcoplasmic reticulum membrane barriers into the cytoplasm of cardiac myocytes for the treatment of cardiac disease and heart failure. The present invention satisfies these needs and provides related advantages as well.

## SUMMARY OF THE INVENTION

It is an advantage of the present invention to provide methods for treatment of heart failure by inhibiting the effect of phospholamban on $Ca^{2+}$ uptake in cardiac tissue.

It is another advantage of the present invention to provide both small peptide complexes and recombinant proteins which function to enhance contractility in failing hearts and reduce blood pressure in individuals with hypertension by inhibiting the interaction between phospholamban and sarcoplasmic reticulum $Ca^{2+}$ ATPase (SERCA2a) within cardiomyocytes.

It is yet another advantage of the present invention to provide for a family of compounds that consist of a transport peptide covalently attached to wild-type, mutant, or truncated PLB.

In a first exemplary embodiment of the present invention, recombinant adenoviruses are engineered which force the expression of wild-type or mutant forms of PLB which have the ability to selectively interrupt the normal inhibitory interaction between PLB and SERCA2a and in turn dominantly activate cardiac contractility.

In a second exemplary embodiment of the present invention, contractilin, a recombinant adenoviral mutant of PLB (K3E/R14E), binds to and imitates phosphorylation of phospholamban. This leads to an activation of the calcium pump of the sarcoplasmic reticulum thus increasing cardiac contractility.

In a third exemplary embodiment of the present invention, a compound consisting of a fusion of 1) a 16-residue transport peptide and 2) a truncated phospholamban protein or similar peptide are transported across the cell membranes in a receptor independent manner. Once inside the cytoplasm of the cardiomyocyte, the truncated phospholamban or similar peptide act as a competitive inhibitor of endogenous phospholamban interactions with SERCA2a.

## BRIEF DESCRIPTION OF DRAWINGS

Figure 1 is an illustration diagraming a working model for the role of the PLB-SERCA2a interaction in the progression of heart failure.

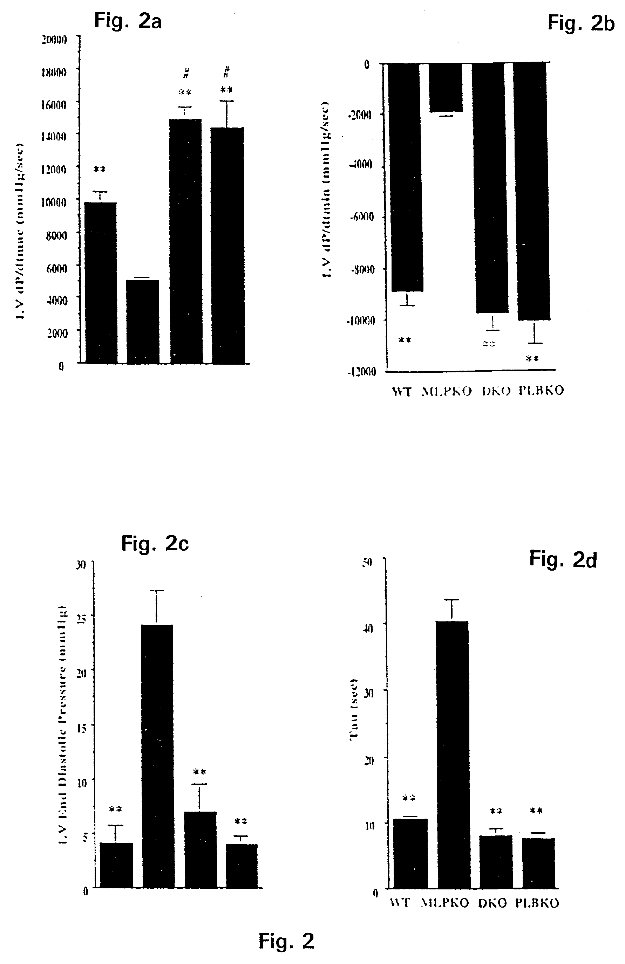

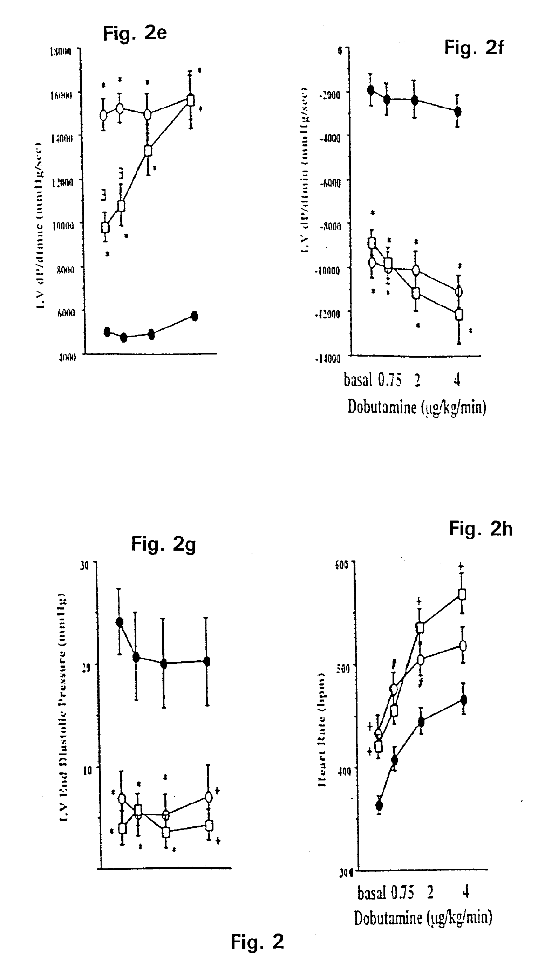

Figure 2 shows the hemodynamic analysis of rescue of in vivo cardiac dysfunction in DKO mice (a-d) and hemodynamic assessment of β-adrenergic response to progressive infusion of dobutamine (e-h), where Figure 2a shows the plot for maximal first derivative of LV pressure, LV dP/dtmax. Figure 2b shows the plot for minimal first derivative of LV pressure, LV dP/dtmin. Figure 2c shows the plot for Lv end diastolic pressure. Figure 2d shows the plot for Tau. Figure 2e shows a graph of maximal first derivative of LV pressure, LV dP/dtmax. Figure 2f shows a graph of minimal first derivative of LV pressure, LV dP/dtmin. Figure 2g shows a graph of Lv end diastolic pressure. Figure 2h shows a graph of heart rate.

Figure 3 shows plotted data from the analysis of rescue of physiological calcium signaling defects in DKO myocytes, where Figure 3a is a series of graphs of representative intracellular $Ca^{2+}$ transient in WT, MLPKO and DKO myocytes. Figure 3b is a bar graph of the amplitude of $Ca^{2+}$ transients. Figure 3c is a bar graph of intracellular diastolic $Ca^{2+}$ concentration. Figure 3d is a bar graph of SR $Ca^{2+}$ content. Figure 3e is the immunoblot of MLP deficiency.

Figure 4a shows a dot blot analysis of rescue of embryonic gene markers of the heart failure phenotype in DKO mice. Figure 4b is a bar graph comparing the relative induction of mRNA for wild-type, MLPKO, and DKO myocytes.

Figure 5 illustrates the inhibition of the interaction between PLB and SERCA2a, where Figure 5a is a series of graphs which plot the length change in myocytes. Figure 5b is a summary of the data of cell length changes.

Figures 6a is a Western blot analysis of myocytes containing the adenovirus transgenes expressing sense PLB (sPLB), antisense PLB (asPLB), E2A, R14E, S16N, and K3E/R14E against monoclonal PLB. Figure 6b is a Western blot showing the results of cell infectivity by PLB, sPLB and K3E/R14E. Figure 6c illustrates the Western blot analysis of PLB infectivity of Sol8 cells.

Figure 7 shows a plot of SR Ca 2 + uptake in homogenates of neonatal rat cardiomyocytes infected with adenovirus expressing the indicated genes.

Figure 8 shows a plot of the data derived from indo 1 fluorescence-facilitated Ca 2 + transients of myocytes.

## DETAILED DESCRIPTION OF THE PREFERRED EMBODIMENT

To directly assess the role $Ca^{2+}$ cycling defects play in the transition to heart failure, cardiomyopathy in muscle-specific LIM protein (MLP)-deficient mice can be reversed by removing the gene that codes for PLB. Mice which harbor an ablation of MLP show many of the phenotypic features of human dilated cardiomyopathy at the molecular, cellular, and physiological levels. A uniform feature of end-stage dilated cardio-myopathy is a marked increase is cardiac wall stress that is accompanied by thinning of the chamber wall and an accompanying decrease in cardiac contractility and relaxation. Because calcium cycling is critical for both cardiac relaxation and contractility, defects in the pathway that control calcium uptake and release from the sarcoplasmic reticulum are prime candidates for driving the progression of heart failure.

The creation of mice which harbor a deficiency in PLB, in addition to MLP, exhibit rescue from all the phenotypic characteristics of human heart failure normally found in MLP single knock-out (MLPKO) mice. To determine whether the functional benefits associated with PLB ablation in MLPKO mice specifically reflect the loss of the direct interaction between PLB and SERCA2a, the applicant of the present invention has engineered point mutations in the PLB gene which interrupt the functional inhibitory interaction between PLB and SERCA2a. Through the creation of recombinant adenoviruses encoding point mutations in PLB, it is demonstrated that progressive defects in excitation-contraction coupling in heart failure are related to the enhanced inhibition of SERCA2a by PLB and that the introduction of phospholamban deficiency into the setting of a transgenic model of cardiac hypertrophy results in rescued cardiac function. These results are independently supported by the fact that MLP-deficient mice harboring a transgene which directs cardiac specific overexpression of SERCA2a also exhibit rescue of the cardiomyopathy phenotype. Taken together, these results provide clear evidence that sarcoplasmic reticulum calcium cycling is critical to the progression of heart failure and points to the critical regulatory role of PLB inhibition of SERCA2a activity in the progression of heart failure. This, in turn, pinpoints the possibility of PLB as a key therapeutic target.

Further study of the MLP-PLB knock-out (DKO) mice indicated that the induction of PLB deficiency in the setting of cardiomyopathic mutation can result in maximal stimulation of cardiac contractile performance. The contractility of the DKO hearts at baseline levels was comparable to the contractility of wild-type hearts following maximal β-adrenergic stimulation. This result suggests that following the removal of the tonic inhibition of SR calcium pump function by PLB, there is essentially a "supra-rescue" in terms of cardiac contractile function of the cardiomyopathic heart. Since PLB is a direct substrate for phosphorylation by both cyclic AMP-dependent protein kinase A and calcium/calmodulin dependent kinase, the regulation of cardiac contractility by cAMP-dependent stimuli may occur via the phosphorylation of PLB, which in turn prevents the inhibitory interaction with SERCA2a.

According to the theory behind the phosphorylation of PLB, the "supra-rescue" of the cardiomyopathic MLP-deficient mice to super-normal levels in the setting of PLB-deficiency might simply reflect the removal of the rate limiting step in the tonic inhibition of cardiac contractility. Consistent with this rationale, studies by Rockman, et al. (Proc. Natl. Acad. Sci. USA 95:7000-7005; 1998) have documented that relief of β-adrenergic desensitization in the MLP-deficient mice can also lead to significant rescue of the dilated cardiomyopathic phenotype. Since PLB is an SR protein that interacts with at least three regulatory components (cAMP-dependent protein kinase, calcium/calmodulin-dependent kinase, and protein phosphatase), it should be determined whether the dominant effect of PLB ablation on improving cardiac contractile performance reflects the chronic interaction of PLB with the SERCA2a or whether this rescue effect is related to the interaction of PLB with other known or novel cardiac proteins.

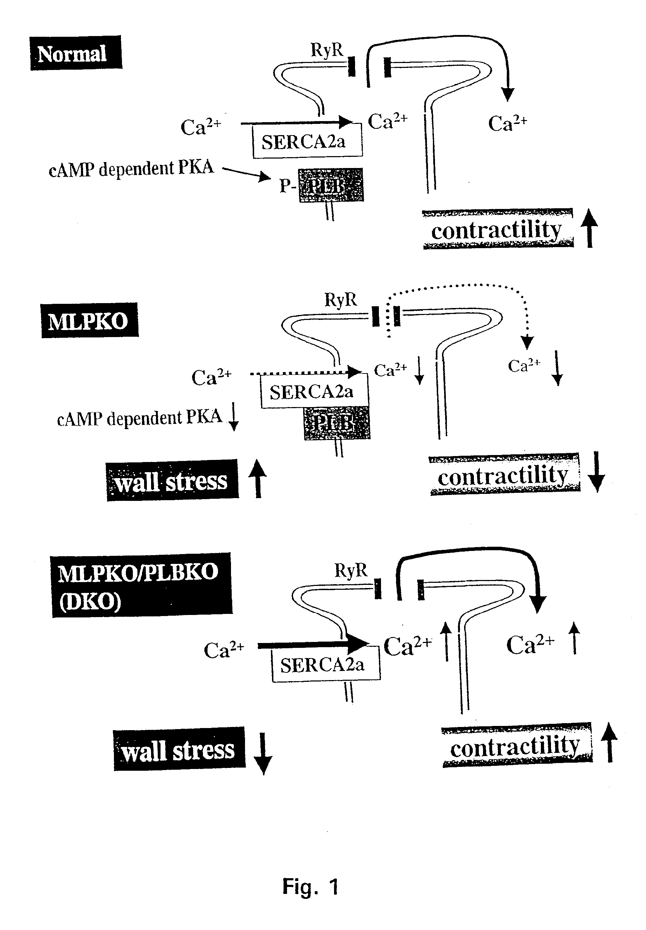

Utilizing recombinant adenoviruses which force the expression of wild-type and mutant forms of PLB, the present invention provides for point mutations in PLB that can selectively interrupt the normal inhibitory interaction between PLB and SERCA2a and can dominantly activate cardiac contractility in cardiac ventricular muscle cells in the absence of catecholamine. Figure 1 outlines the mechanism responsible for the rescue effect observed in the DKO mice. Both PLB and MLP are muscle-specific proteins, and as such, there must be a muscle cell autonomous pathway that is required for the progression and the rescue of the phenotype, as opposed to extrinsic stress signals that promote or suppress myocyte survival pathways. Since PLB and MLP do not directly interact at the protein level, eliminating direct interaction between PLB and MLP as the basis for rescue, there must be a physiological as opposed to biochemical regulatory pathway that links the PLB regulatory pathways with the onset of dilated cardiomyopathy. As shown in Figure 1, in the normal heart, SR-calcium stores are maintained through the activity of the SERCA2a which leads to an uptake of calcium into the SR and consequent maintenance of normal cardiac relaxation and reduction in wall stress. Subsequently, SR release of calcium, via the calcium release channel, results in normal quantal calcium release and the consequent activation of the cardiac myofilaments leading to myocardial contraction. Accordingly, enhanced $Ca^{2+}$ content in the SR leads to an enhanced $Ca^{2+}$ release with a corresponding increase in cardiac contractility.

The activity of SERCA2a is regulated by the inhibitory effects of the direct interaction of PLB with SERCA2a, which can be relieved by the cAMP-dependent phosphorylation of PLB following the delivery of β-adrenergic stimuli. In the setting of heart failure, there is a relative decrease in SERCA2a function due to an inhibitory effect of PLB that arises due to blunted β-adrenergic responsiveness. As a result, there is less phosphorylation of PLB and a constitutive inhibition of SERCA2a, via chronic interaction with PLB, leading to a relative decrease is SR calcium content versus normal levels. This decrease in calcium stores is translated into a decrease in the quantal release of calcium through the calcium release channel and a consequent decrease in the single cell calcium transients and *in vivo* cardiac contractility. In the DKO mice, the inhibitory effect of PLB is removed, as shown in Figure 1, thereby relieving the system from the downstream inhibitory effects of PLB on the SR calcium pump, resulting in maintenance of SR calcium uptake and reduction of wall stress towards normal levels. At the same time, this increase in SR calcium content results in maintenance of normal calcium quantal release, thereby leading to maintenance of normal contractility and relaxation.

*Muscle-specific LIM protein knock-out and Double knock-out mice*

Tests of the present invention were conducted using a double knock-out (DKO) mouse model which harbors homozygous ablation of two independent muscle specific genes. For this strategy, PLB knock-out (PLBKO) mice are mated into the background of MLP knock-out (MLPKO) mice which harbor molecular, structural and physiological features of the complex *in vivo* heart failure phenotype of dilated cardiomyopathy. The F3 generation of these mice are used for the actual experimentation to eliminate any potential background effects from either the PLBKO or MLPKO line on the observed cardiac phenotype of the DKO line.

MLPKO mice display a marked increase in heart/body weight ratio (6.34 $\pm$ 0.22 mg/g, n=8) versus age and gender matched wild-type mice (4.60 $\pm$ 0.21 mg/g, n = 7; p<0.001). The heart/body weight ratio in DKO mice (5.13 $\pm$ 0.19 mg/g, n = 9) is significantly smaller than that of MLPKO mice (p<0.01) and is not statistically different from wild-type. To evaluate whether the decreased heart weight in DKO mice is associated with amelioration of the disrupted cytoskeletal phenotype observed in MLPKO mice, electron microscopic analysis is made of hearts from MLPKO and DKO littermates. Ablation of PLB in the background of MLP $^{-/-}$ rescues the wide spectrum of ultrastructural defects originally observed in the MLP deficient hearts, including myofibrillar disarray and massive fibrosis. These data suggest that ablation of PLB prevents not only the increase in total heart mass, but also prevents the disorganization of cardiomyocyte cytoarchitecture and fibrosis in MLPKO cardiomyopathic mice.

To evaluate whether the marked decreases observed in *in vivo* global cardiac function is rescued in DKO mice, echocardiography is performed with age-matched littermates. As noted in Table 1, prevention of ventricular dilation is confirmed in DKO mice. MLPKO mice have enlarged cardiac chambers, as revealed by increased left ventricular end-diastolic dimensions (LVEDD) and end-systolic dimensions (LVESD), whereas DKO mice have LVEDD in the normal range. The percent fractional shortening (%FS) and mean velocity of circumferential fiber shortening (mean Vcf), indicators of systolic cardiac function, are improved in age-matched DKO littermates, also noted in Table 1. When compared to non-littermate wild-type mice as controls, most of the echocardiographic data in DKO mice are similar to those in wild-type mice, although %FS is slightly decreased in DKO mice. Furthermore, cardiac function of DKO mice remain in the normal range beyond 6 months of age (n = 2). Despite the reduction of chamber dilation, there appears to be some hypertrophy in DKO hearts. The ratio of LVEDD to LV posterior wall thickness is markedly decreased in DKO mice, indicating that the wall stress of DKO mice is reduced in comparison to MLPKO or wild-type mice. These results indicate that global cardiac function of DKO mice is preserved in the range comparable to the parameters of control hearts. It should be noted that mice which are heterozygous for PLB deficiency display an intermediate level of functional rescue versus the MLPKO and DKO mice, suggesting that partial ablation of PLB can lead to significant functional improvement of the heart failure phenotype in MLPKO mice.

Table 1

Echocardiographic assessment

| | MLPKO n=12 | MLPKO/PLBhet n=12 | DKO n=10 | wild-type n=10 |
|---|---|---|---|---|
| LVEDD (mm) | 4.87±0.14 | 4.10±0.15*** | 3.75±0.12*** | 3.89±0.11*** |
| LVESD (mm) | 3.94±0.18 | 3.06±0.22***† | 2.60±0.10*** | 2.44±0.07*** |
| FS(%) | 19.4±1.7 | 26.2±3.2*‡ | 30.6±1.8***† | 37.2±1.4*** |
| SEPth (mm) | 0.68±0.05 | 0.86±0.05*‡ | 0.83±0.05*‡ | 0.64±0.01 |
| Pwth (mm) | 0.67±0.05 | 0.86±0.05*‡ | 0.81±0.04*† | 0.64±0.06 |
| LVEDD/ Pwth(mm/mm) | 7.47±0.30 | 5.00±0.47*** | 4.74±0.30*** | 6.14±0.18* |
| mean Vcf (circ/s) | 3.38±0.33 | 4.81±0.73# | 6.10±0.41***† | 4.41±0.61 |
| mean Vcfc (circ/s) | 1.41±0.17 | 1.96±0.27* | 2.37±0.13*** | 2.23±0.10** |
| Heart Rate (beats/min) | 351±15 | 350±15§ | 396±13§ | 239±14*** |
| Age (days) | 65.3±2.5 | 65.5±4.5‡ | 67.4±1.9‡ | 80.5±1.2** |
| Body Weight (g) | 26.7±1.3 | 26.8±0.9§ | 25.0±1.3§ | 36.9±2.2*** |

MLPKO vs. MLPKO/PLBhet or DKO or wild-type; *: p<0.05, **: p<0.01, ***: p<0.001
wild-type vs. MLPKO/PLBhet or DKO; t: p<0.05, t: p<0.01, §: p<0.001
MLPKO/ PLBhet vs. DKO; #: p<0.05

MLPKO mice have demonstrated a marked blunting of β-adrenergic responsiveness and decreased adenyl cyclase activity. Ablation of PLB by homologous recombination in mice can augment cardiac contractile performance to a level comparable to that with maximal β-adrenergic stimulation of the normal heart. To confirm that the ablation of PLB can reverse the hemodynamic defects and marked β-adrenergic receptor desensitization associated with dilated cardiomyopathy, anesthetized mice are cardiac catheterized and assessed.

Several independent hemodynamic parameters document the rescue of the severe cardiac dysfunction with circulatory congestion to normal levels in the DKO mice. LV contractility (assessed by LV dP/dtmax) and relaxation (assessed by LV dP/dtmin) at baseline appears to be higher than that of wild-type mice and is comparable to PLBKO mice, as is shown in Figures 2a and 2b. Ablation of PLB reverses the markedly elevated LV end diastolic pressure observed in the MLPKO cardiomyopathic mice, as seen in Figure 2c.

Analysis of Figure 2d illustrates that Tau, an indicator of LV relaxation and diastolic function, are also normalized in the DKO mice, which is consistent with an improvement in wall stress. These data suggest that ablation of PLB can rescue both the systolic and diastolic dysfunction in the cardiomyopathic MLPKO mice. The blunted responses of LV dP/dtmax and LV dP/dtmin to β-adrenergic stimulation is observed in MLPKO mice, as shown in Figures 2e and 2f, indicating the presence of severe β-adrenergic desensitization in MLPKO mice. There is no stimulation of cardiac contractility (LV dP/dtmax) and relaxation (LV dP/dtmin) by dobutamine in DKO mice, as is again shown in Figures 2e and 2f. These parameters are already stimulated to their maximal levels under basal conditions in the DKO hearts.

After maximum stimulation of wild-type hearts by dobutamine, LV dP/dtmax and LV dP/dtmin are indistinguishable from these parameters in the DKO mice in the absence of any catecholamine stimulation. This evidence confirms that the interaction of PLB and SERCA2a suppresses cardiac contractility in both normal and myopathic hearts, and that inhibiting this interaction may exert a dominant effect on maximizing cardiac performance in the absence of any catecholamine stimulation. Figure 2h shows that chronotropic responsiveness to dobutamine is preserved in both DKO mice and MLPKO mice, thereby documenting the specificity of the β-adrenergic response to ventricular myocytes versus pacemaker cell types.

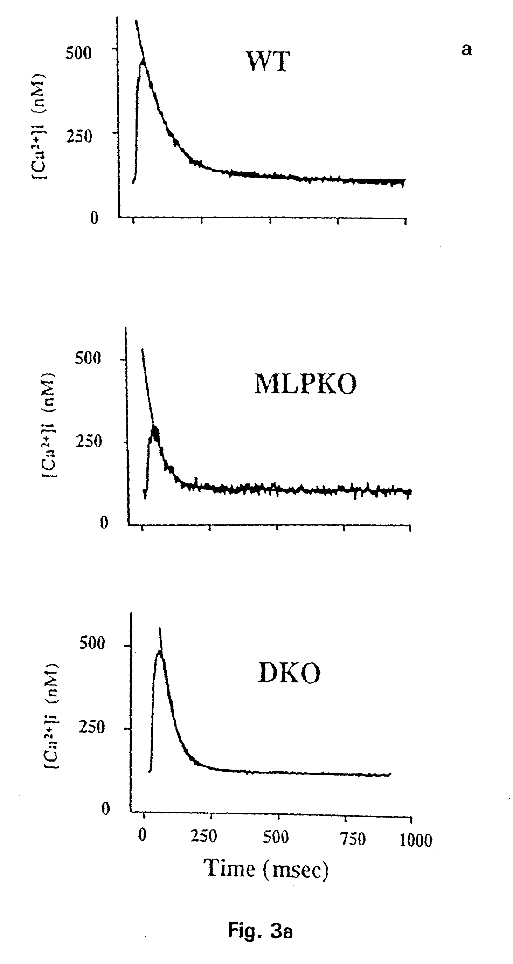

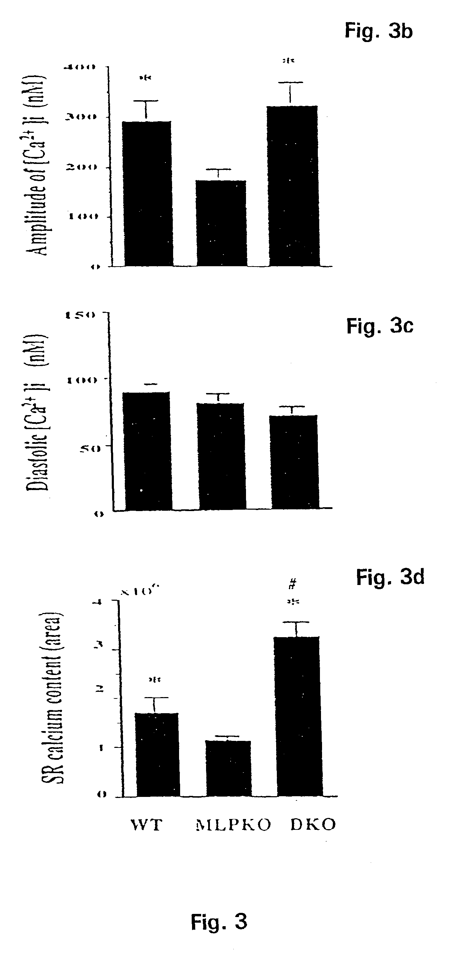

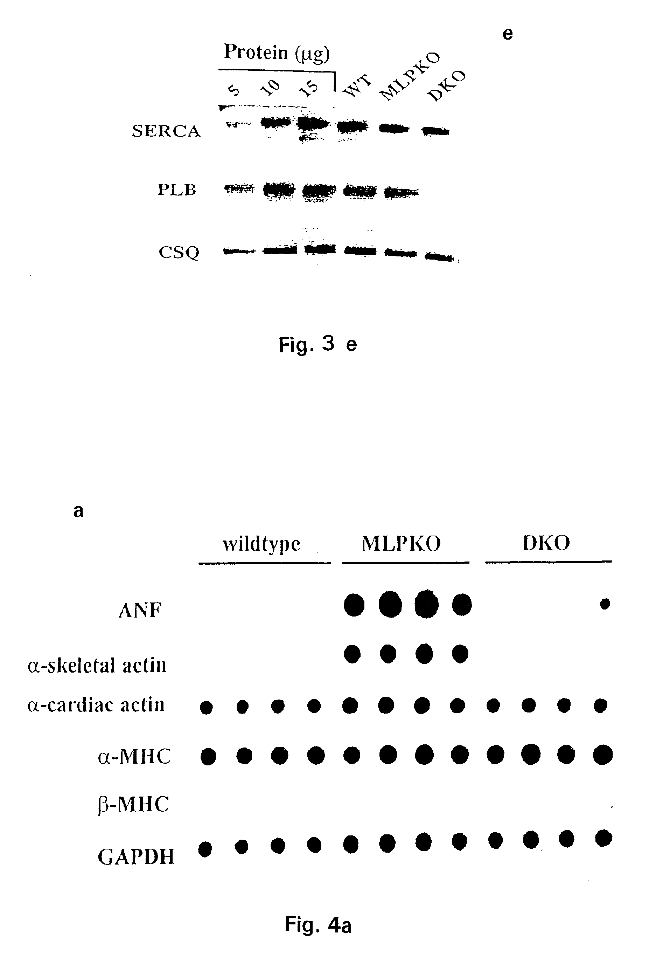

To determine the mechanisms responsible for the rescue of *in vivo* cardiac function in DKO mice, several independent parameters of $Ca^{2+}$ signaling are assessed. It is apparent that altered $Ca^{2+}$ homeostasis in DKO mice leads to the hemodynamic changes, intracellular $Ca^{2+}$ transients and the expression of proteins related to $Ca^{2+}$ cycling in the SR. MLPKO myocytes exhibit an attenuated amplitude of $Ca^{2+}$ transients with normal levels of diastolic $Ca^{2+}$ concentration, as is show in Figures 3a-c. The rate of decay is slightly accelerated in MLPKO mice which suggests that a compensatory mechanism is operative during the end of $Ca^{2+}$ uptake in MLPKO mice. Ablation of PLB is associated with a shortened duration of the $Ca^{2+}$ transient, faster decay and preserved amplitude. Figure 3d shows that SR $Ca^{2+}$ content is significantly decreased in MLPKO mice and increased in DKO mice as compared to wild-type mice. Quantitative immunoblotting, shown in Figure 3e, reveals that MLP deficiency is not associated with any significant alterations in protein levels of SERCA2a, PLB and calsequestrin, suggesting that the defects of $Ca^{2+}$ cycling in MLPKO mice is based upon functional impairment of EC coupling, as opposed to simply reflecting decreases in the protein levels of either SERCA2a or phospholamban.

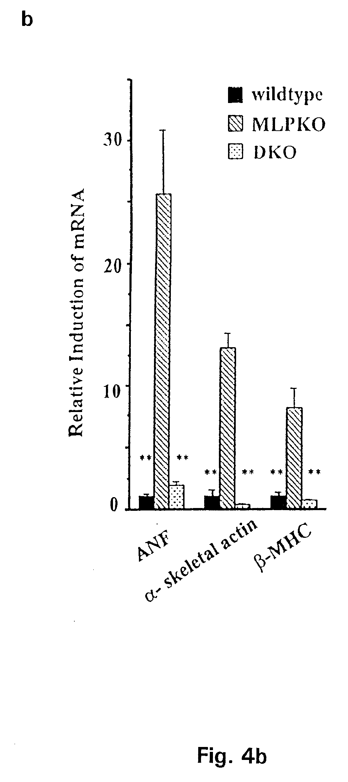

One of the characteristic features of heart failure is the reactivation of an embryonic gene program which may contribute to a compensatory response to an increased hemodynamic load. To confirm that hemodynamic improvement in DKO mice is accompanied by amelioration of changes at the transcriptional level, the expression of ANF, α-skeletal actin, and β-MHC mRNAs, well established embryonic markers for heart failure, is examined. As shown in Figures 4a and 4b, ventricles of MLPKO mice display a 26-fold increase in ANF, a 13-fold increase in α-skeletal actin and an 8-fold enhancement of β-MHC mRNAs. DKO mice exhibits only a 1.9-fold increase in ANF and no significant increase in α-skeletal actin or β-MHC mRNAs. Thus, ablation of PLB largely suppresses induction of the embryonic gene program in MLPKO mice.

The aforementioned studies indicate that the ablation of PLB can rescue independent parameters of heart failure and associated defects in cardiac contractility. To define the mechanism of the rescue effect, it is necessary to assess whether the chronic interaction of PLB and SERCA2a is in fact limiting for cardiac contractility in both normal and myopathic hearts. The "supra-rescue" of basal cardiac function in the DKO mice to levels comparable to those in wild-type mice following maximal catecholamine stimulation suggests that inhibition of this interaction may exert a dominant effect to maximize cardiac performance in the absence of any catecholamine stimulation.

*Recombinant Adenoviral transgene mutants of PLB*

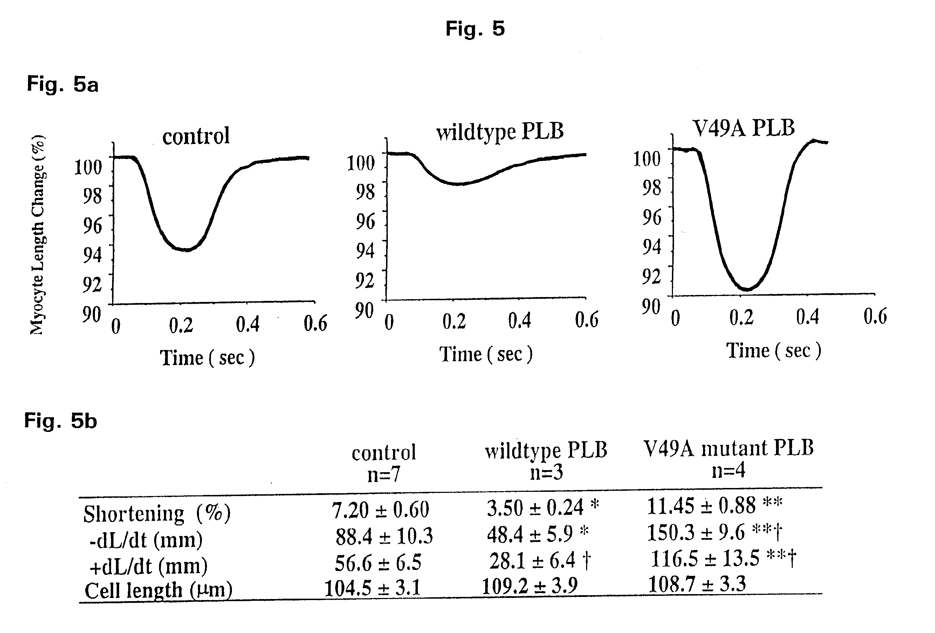

Using the knowledge that certain amino acid residues of PLB are required to maintain its inhibitory effects on SERCA2a, several single point mutations of PLB, V49A (Seq. ID. No. 2), E2A (Seq. ID, No. 3), R14E (Seq. ID. No. 4), S16N (Seq. ID. No. 5),a double point mutation of PLB, K3E/R14E (Seq. ID. No. 6) and a sense and antisense PLB (Seq. ID. No. 1) transgene can be engineered in order to disrupt the inhibitory effects of PLB on SERCA2a. Using recombinant adenoviruses for *in vivo* murine cardiac gene transfer, myocytes which overexpresses V49A, one of the single point mutations in PLB, exhibit an increase in contractility, while myocytes which overexpress the wild-type PLB exhibit a decrease in contractility when compared to non-infected myocytes, as is documented in Figure 5. It can be concluded that the feasibility and utility of interfering with the interaction between the SERCA2a and PLB is clearly documented. The PLB-SERCA2a interaction appears to be the rate limiting step for establishing the set point of basal cardiac contractility and relaxation in vivo, and the disruption of this interaction can thereby short circuit the β-adrenergic pathway.

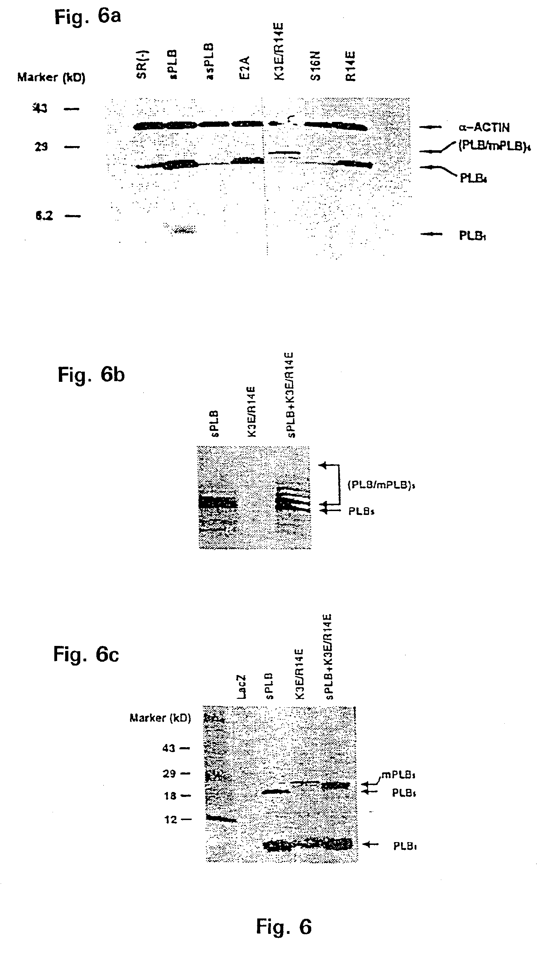

Additional Western blot analysis of myocytes containing the adenovirus transgenes expressing sense PLB (sPLB), antisense PLB (asPLB), E2A, R 14E, S 16N, and K3E/R14E against monoclonal PLB antibody (Affinity BioReagents) is shown in Figure 6a. Quantification of PLB protein content, normalized to α-actin for loading variance and compared with an adenovirus/ SR control lacking the transgene, shows that sPLB, E2A, and R14E mutants increase PLB protein level by 150% ($PLB_5$ + $PLB_1$), 72 %, and 57%, respectively. In contrast, asPLB and S16N results in 54% and 33% decrease in PLB protein content within the myocytes. The introduction of K3E/R14E transgene infection of myocytes leads to a formation of a distinct pattern of pentamer PLB. Multiple PLB bands appear in addition to $PLB_5$ (the pentamer). This is accompanied by a reduced abundance of $PLB_5$ in comparison with the control.

The nature of the Western blot banding pattern is further evaluated by substituting PLB-deficient Sol8 cells in place of cardiac myocytes. Sol8 cells are infected with the recombinant adenovirus expressing either the transgene sPLB or K3E/R14E alone or in combination. As seen in Figure 6b, the Western blot shows that the monoclonal PLB antibody detects PLB in cells infected by sPLB but fails to detect K3E/R14E. Infection of Sol8 cells with a combination of the adenoviral transgenes results in formation of multiple bands of PLB. Moreover, the PLB pentamer decreases in abundance simultaneously with the appearance of the upper bands. It is well established that PLB interacts with and inhibits SERCA2a predominantly as a monomer that exists in equilibrium with the noninhibitory pentamer. Based on this knowledge, the heteropentamer of K3E/R14E and wild-type PLB might be more stable that the homopentamer of wild-type PLB. Therefore, the dissociation of the heteropentamer into monomers, which results in inhibition of SERCA2a, is disfavored. K3E/R14E interacts with endogenous PLB and forms such a complex, accompanied by a decrease in homopentamer formation. In as much, the monomer K3E/R14E may act as a noninhibitory competitor for endogenous wild-type PLB by blocking SERCA2a-PLB interaction sites.

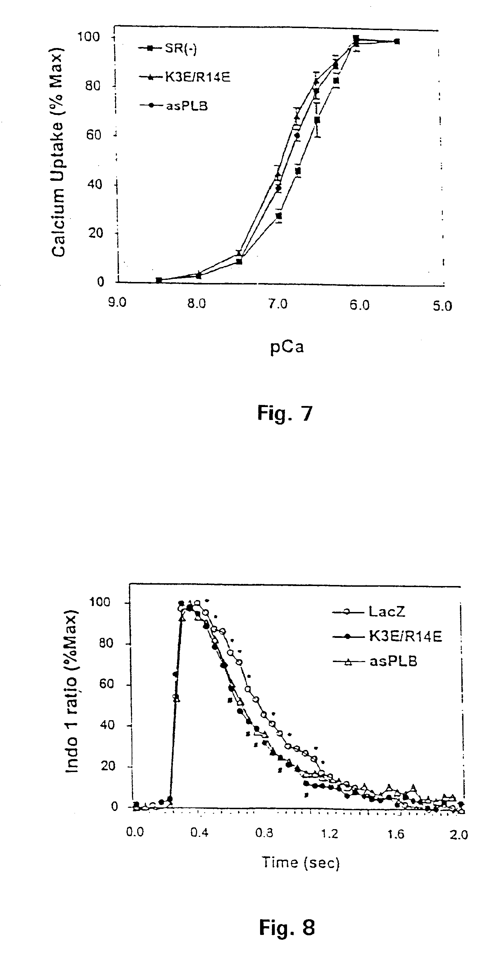

The effects of mutant and antisense PLB on SERCA2a is further evaluated by determination of the SR calcium uptake activity. The initial rate of $Ca^{2+}$ uptake by the SR measured at varying $Ca^{2+}$ concentrations reflects the activity of SERCA2a. As shown in Figure 7, neonatal rat myocytes infected with the recombinant adenovirus transgenes K3E/R14E and asPLB show a decrease in the concentration of $Ca^{2+}$ needed by SERCA2a for the same activity compared with the non-transgene control, indicating a stimulation of SERCA2a activity. The $EC_{50}$ s of $Ca^{2+}$ concentration at which the uptake activity is half-maximal are, (in $\mu$mol/L), $0.20 \pm 0.02$ for the non-transgene control (SR-), $0.11 \pm 0.01$ for K3E/R14E, and $0.13 \pm 0.01$ for asPLB. The effects of K2E/R14E and asPLB on SERCA2a are also examined in adult rat myocytes. The adenoviral transgene K3E/R14E lowers the $EC_{50}$ significantly (by 36%), whereas the change as a result of asPLB infection is not within statistical significance. This apparent discrepancy in the effects between neonatal and adult cardiac myocytes is possibly related to the different abundances of PLB in myocytes at different developmental stages. PLB is nearly twice as abundant in adult as in neonatal myocardium.

To further examine the effects of K3E/R14E and asPLB on SERCA2a, intracellular $Ca^{2+}$ transients in neonatal myocytes are measured by use of the indo 1 fluorescence indicator. Indo 1 ratiometric data which is obtained from each of the experimental conditions is normalized to the respective maximum and minimum of each contractile $Ca^{2+}$ transient and is then aligned and averaged. As shown in Figure 8, the decay curves of K3E/R14E and asPLB are displaced to the left of the LacZ control. Furthermore, for most of the diastolic time points, K3E/R14E is significantly different from LacZ, whereas at several diastolic time points, asPLB is also significantly different from LacZ. The half-times for decay ($RT_{50}$) for LacZ, K3E/R14E, and asPLB are determined to be 0.28 seconds (100%), 0.20 seconds (73%), and 0.22 seconds (79%), respectively. The values for K3E/R14E (73%) and asPLB (79%) are significantly different ($p<0.05$) from the values obtained from the LacZ expressing virus.

In addition to generating adenoviral transgenes using various point mutations of PLB, or the sense or antisense sequences of PLB, antibodies raised against PLB peptide and then expressed as RNA can also be inserted into the adenoviral vector. To raise polyclonal PLB antibody ("contractilin", or chicken antibody peptides with hyperactive regions), a chicken is repeatedly immunized with PLB peptide which represents amino acids 3 to 19 of the cytoplasmic domain. After three rounds of booster immunization, administered at 15, 42 and 54 days, total IgY is purified from the egg yolk, using a commercially available purification system (EGGstract IgY Purification System - Promega). Upon confirmation of a positive immune response, lymphocytes from the spleen and bone marrow are harvested. RNA, in the form of the hypervariable regions from both antibody light and heavy chain is obtained from these cells and amplified by RT-PCR, the method of which is well known.

The amplified and purified hypervariable region RNA is then fused to a single cDNA (Seq. ID. No. 9) and subsequently cloned in frame into a plasmid vector, coding for a phage surface protein. Using standard phage display technique, phages which express the immune library of the chicken are selected by their positive response to the PLB peptide. After a series of enrichment for phages which specifically bind PLB, 20 clones are selected for ELISA. The resulting 5 best binders are then analyzed using a radioactive $Ca^{2+}$ transport assay. The two best activators of SR $Ca^{2+}$ transport are further analyzed. Both clones are found to dramatically stimulate the rate of $Ca^{2+}$ transport into the SR.

To demonstrate that the recombinant protein, which has been generated from contractilin (the PLB antibody), can also function inside a living cell, an adenoviral vector expressing contractilin is constructed. Western blot analysis of neonatal and adult rat cardiomyocytes, infected with the adenoviral transgene, shows that contractilin can be expressed in heart cells. Radioactive $Ca^{2+}$ transport analysis indicate that, as with the mutant and antisense PLB, contractilin accelerates cytoplasmic $Ca^{2+}$ removal.

In a complementary approach, plasmid transfection rather than adenoviral transfection is used for gene delivery. It is found that K3E/R14E- and asPLB- transfected myocytes, as monitored by co-transfected green fluorescence protein, exhibits 43% ($p<0.05$) and 9% ($p<0.1$) decreases in $RT_{50}$, respectively, relative to adenoviral vector transfected cells. Thus, introducing K3E/R14E and asPLB into the cardiac myocytes by either the adenovirus or co-transfection technique reduces the duration of the diastolic $Ca^{2+}$ transients. These results would seem to mirror the findings of MLPKO versus DKO mice where variation in $Ca^{2+}$ transients confirm that ablation of PLB is associated with a shortened duration of $Ca^{2+}$ transient, faster decay, and preserved amplitude. Taken together, these data confirm that K3E/R14E and asPLB stimulate the SERCA2a activity, which results if faster $Ca^{2+}$ transients in myocytes.

To determine whether the enhanced SERCA2a activity and accelerated $Ca^{2+}$ transients, as a result of the PLB mutants, lead to change in contractile behavior, edge detection is used to analyze myocyte contractility. Adult rabbit myocytes are infected with the adenoviral transgenes of LacZ, K3E/R14E, or asPLB. After a three day incubation period, there is a significant difference in the number of spontaneously contracting cells between the different groups (LacZ< <asPLB< K3E/R14E). Table 2 provides the effects of K3E/R14E and asPLB on myocyte contractility. As shown in the table, compared with the LacZ control, K3E/R14E increases fractional shortening by 74%, which is accompanied by a 25% decrease in $RT_{50}$ and a 115% increase in + dL/dt. When the myocyte contractility is examined after asPLB infection, it is found that the fractional shortening of the myocytes increases significantly, by 57%, whereas the changes in $RT_{50}$ and +dL/dt are not significant.

Table 2

|  | LacZ (n=33) | K3E/R14E (n=29) | asPLB (n=30) |
|---|---|---|---|
| +dL/dt ($\mu$m/s) | $11.7 \pm 1.9$ | $25.1 \pm 1.6^*$ | $18.4 \pm 2.0^*$ |
| $RT_{50}$ (ms) | $539.0 \pm 27.0$ | $402.0 \pm 19.0^*$ | $483.0 \pm 29.0^{***}$ |
| Shortening (%) | $6.2 \pm 0.5$ | $10.8 \pm 0.5^*$ | $8.0 \pm 0.6^{***}$ |

\*: $p < 0.05$
\*\*: $p < 0.1$

The resulting data show that the increase in SERCA2a activity translates into an accelerated relaxation of the myocytes. K3E/R14E-infected myocytes display an enhanced fractional shortening, which suggests an increase in SR loads of $Ca^{2+}$ due to the enhancement of SERCA2a activity. Further, K3E/R14E infection increases the number of spontaneously contracting myocytes, a phenomenon most likely associated with the increased amount of oscillating $Ca^{2+}$ due to the elevated SR loading of $Ca^{2+}$. Taken together, these data show that K3E/R14E affects endogenous wild-type PLB in a way that significantly reduces its inhibition of SERCA2a and thus has a dominant inhibitory effect over wild-type PLB.

*Peptide-based therapeutic for inhibition of PLB activity*

Still further, the present invention provides for a peptide based therapeutic for the inhibition of phospholamban activity and a mode of delivery for such a therapeutic, based on the finding that PLB function can be inhibited in a dominant negative manner by overwhelming endogenous PLB with mutant PLB molecules, and that this inhibition leads to improved function in failing hearts.

For a therapeutic agent, such as an inhibitor of the PLB-SERCA2a interaction, to effect a target cell system, it must have a means for internalization through the cell membrane into the cytoplasm. The mode of transfer of the inhibitor can be by way of either a transport or penetratin based PLB peptide or it can also include adenoviral or lipid vesicle based transfer. For this purpose, a compound consisting of a fusion of a transport peptide and a PLB protein molecule is constructed. The transport peptide comprises a 16-residue from the sequence for antennapedia, a Drosophila transcription factor protein. The second peptide of the complex can be a truncated sequence of PLB protein. Further therapeutic benefits can be achieved using peptides that correspond to native PLB protein as well as a mutant or truncated form of PLB protein.

One beneficial function of the transport peptide-PLB complex is the inhibition of the interaction between PLB and SERCA2a within cardiomyocytes, resulting in enhanced contractility in diseased hearts. The present invention may also inhibit the interaction of PLB with SERCA2a within the smooth muscle layer surrounding the arteries/arterioles of the circulatory system which would result in vasodilation and reduced blood pressure. Thus, there is a two-fold benefit in the treatment of heart disease, the first is enhanced cardiac contractility in failing hearts, the other is the reduction of blood pressure in individuals with hypertension. It is also predicted that there will be the inhibition of PLB interactions with SERCA proteins of other cell types, such as the SERCA1-PLB interaction in nervous tissue.

The introduction of the molecule into the blood stream feeding the heart can is best achieved using a catheter located in the coronary artery. When the molecule is present in the extracellular environment surrounding a cardiomyocyte it rapidly enters the cardiomyocyte and inhibits the association of PLB with SERCA2a. The translocation function is attributable to the transport peptide which exhibits the ability to rapidly translocate itself and the attached "cargo" peptides across the cell membranes in a receptor independent manner. Once inside the cytoplasm of the cardiomyocyte, the PLB fragment will act as a competitive inhibitor of endogenous PLB interactions with SERCA2a.

In the absence of PLB inhibition by association, SERCA2a more efficiently pumps Ca 2 + into the SR, thereby increasing the cardiomyocytes ability to contract more strongly and rapidly. Stronger cardiomyocyte contractility translates to more powerful heart contractility. *In vivo,* the present invention could act as a treatment for heart failure and is most easily administered and most effective in patients whose hearts require, or already have implanted, a left-ventricular assist device (LVAD).

While residues 43 to 58 of Antennapedia is a well characterized translocation peptide, and works well in the present invention, the present invention is not restricted to this method of transport. Other potential methods of transfer include the use of an 8-branched polylysine backbone to link the transport and cargo peptide, but it is not limited to this multibranched structure. A compound consisting of one target peptide attached to one PLB peptide, as one long peptide, has also been explored. Still further, a number of DNA constructs for producing hexahistidine (H6) tagged penetratin and penetratin-PLB recombinant proteins in bacteria have been undertaken. The penetratin peptides were engineered to be on either the amino or carboxy terminal end of the protein.

It has been shown that the cytoplasmic fragment of PLB has as strong a binding affinity for the cytoplasmic portion of SERCA2a as the whole PLB molecule. Therefore, once the transport-PLB molecule is inside the cytoplasm of the cardiomyocyte, the PLB fragment is predicted to act as a competitive inhibitor of endogenous PLB interaction with SERCA2a.

This form of treatment is suitable for the patient who is suffering from severe decreased cardiac pump function, refractory to medical therapy, and requiring mechanical assistant devices while waiting for heart transplantation. In addition, the underlined molecular mechanisms for the dominant negative function of the PLB mutants can be used in the design and implementation of the high-throughput screening strategies for inhibitory small molecules.

The following examples are intended to illustrate but not limit the present invention.

### EXAMPLE 1

Creation of knock-out mouse lines for echocardiography

In order to analyze the structural and physiological features of the complex *in vivo* heart failure phenotype of dilated cardiomyopathy, several lines of knock-out mice were created. conducted using a double knock-out (DKO) mouse model which harbors homozygous ablation of two independent muscle specific genes. For this strategy, PLB $^{-/-}$ (phospholamban deficient) homozygous mice were mated with MLP $^{-/-}$ (muscle- specific LIM protein) homozygous mice. The F1 pups generated from an MLP$^{-/-}$ x PLB$^{-/-}$ homozygote cross were then mated to create the MLP$^{+/-}$, PLB$^{+/-}$ double heterozygote genotype. F2 offspring were generated from a MLP$^{+/-}$ / PLB$^{+/-}$ double heterozygote mating, thereby creating mice that were homozygous for the mutant MLP allele and heterozygous for the mutant PLB allele or that were MLP wild-type and heterozygous for the mutant PLB allele. The F3 offspring were generated from a MLP$^{-/-}$ / PLB$^{+/-}$ matings to generate MLP$^{-/-}$ / PLB$^{-/-}$ (DKO), MLP$^{-/-}$ /PLB$^{+/+}$ (MLPKO) and MLP$^{+/+}$ / PLB$^{-/-}$ (MLPKO/ PLBhet) littermates or from a MLP$^{+/+}$ / PLB$^{+/-}$ matings to generate MLP$^{+/+}$ / PLB$^{-/-}$ (PLBKO), MLP$^{+/+}$ /PLB$^{+/+}$ (wild-type) and MLP$^{+/+}$ / PLB$^{+/-}$ (PLBhet) littermates. The genotype of the gene-targeted crosses were determined by PCR or genomic DNA isolated from tail biopsies.

To evaluate the hemodynamic properties of the various knock-out mouse lines, cardiac catherization and echocardiography was performed on subjects anesthetized with either Avertin (2.5%, 20 $\mu$l/kg body weight) or xylazine (0.005 mg/g) and ketamine (0.1 mg/g). Transthoracic M-mode echocardiographic tracings indicated that MLPKO mice had chamber dilation with reduced wall motion, indicating depressed cardiac function and increased wall stress, whereas chamber size and cardiac function are normal in the DKO mice. Baseline parameters for wild-type (WT), n = 7, MLPKO, n = 8, DKO, n = 9, and PLBKO, n = 5 are shown in Figures 2a-d. Data were expressed as mean $\pm$SEM. MLPKO versus other groups; *p<0.5, **p<0.001, WT vs. DKO; #p<0.01. In Figures2e-h, hemodynamic assessment was made of β-adrenergic responsiveness to progressive infusion of dobutamine, where WT ($\square$), n = 7, MLPKO (●), n = 8, and DKO (○), n-9, mice. MLPKO vs WT or DKO; #p<0.05, $^{+}$p<0.01, *p<0.001, WT vs DKO; ∋p<0.01.

### EXAMPLE 2

Calcium transient analysis

To evaluate the effect of inhibition of PLB on SR calcium content and calcium transients, myocytes were isolated from the right ventricular wall of the wild-type or knock-out mice. To monitor the changes in intracellular calcium, the isolated myocytes were incubated with a calcium sensitive dye, fluo-3-AM (1 $\mu$g/ml), for 30 minutes at room temperature. The myocytes were then transferred to a tissue chamber on the stage of an inverted microscope and continuously stimulated at a rate of 1 Hz to maintain a consistent degree of SR calcium loading. To measure cellular fluorescence, the myocytes were illuminated with an excitation wavelength of 480 nm. Any changes in fluorescence were monitored at 510 nm using a microfluorometer (FM-1000; Solamere Technologies) and digitally recorded for later analysis using Cellsoft (D. Bergman; University of Calgary) software. Fluorescence values were calibrated using the equation:

$$[Ca^{2+}]i = K_D(F\text{-}Fmin)/(Fmax\text{-}F) \qquad (1)$$

with an assumed $K_D$ of 864 nM, where F are the experimentally derived fluorescence values. Fmax was determined by adding 10 $\mu$M ionomycin to the superfusion solution and Fmin was determined by adding 4 mM $MnCl_2$ to the superfusion solution for each myocyte.

SR calcium content of the isolated myocytes was assessed using a standard caffeine pulse protocol. Following stable recordings of calcium transients, a 20 second pulse of 10 mM caffeine was applied to the myocyte. This protocol resulted in a rapid caffeine-induced transient which slowly decayed back to baseline values. The SR calcium content was defined as the integrated area of this caffeine-induced calcium transient. Figure 3a illustrates the representative intracellular calcium transient in the WT, MLPKO and DKO myocytes. MLPKO myocytes exhibited an attenuated amplitude of calcium transients with normal levels of diastolic calcium concentration. DKO myocytes displayed the calcium transient with a shortened duration, faster decay, and preserved amplitude. As shown in Figure 3b, the amplitude of calcium transient was significantly attenuated in MLPKO myocytes and was restored in DKO myocytes. Figure 3c shows that intracellular diastolic calcium concentration was not different among the three different groups of myocytes. In Figure 3d, SR calcium content was significantly decreased in MLPKO mice and increased in DKO mice when compared to WT mice. In Figure 3e, representative quantitative immunoblotting revealed that MLP deficiency was not associated with any significant alterations in the protein levels of SERCA2a, PLB, and calsequestrin.

## EXAMPLE 3

Construction of mutant PLB adenovirus and gene transfer

I.M.A.G.E. consortium cDNA clones encoding human PLB were available through Genome System, Inc. The DNA fragment harboring the entire coding sequence of PLB was subcloned into pBluescriptll KS vector, as well known E. coli cloning vector (ATCC accession no. 87047). A sense mutation (Val49Ala) was introduced using a PCR based mutagenesis system commercially available from Stratagene. Recombinant adenovirus expressing wild-type and mutant human PLB was generated by homologous recombination between plasmid pJM17 and a shuttle plasmid containing RSV promoter and SV40 poly A sequences. The concentrated virus preparation were tittered using a standard protocol. The efficient *in vivo* cardiac gene transfer was performed by injecting the adenovirus vectors into 1 day old neonatal mouse heart. The myocytes were isolated 4 weeks after injection into the mouse hearts and cell shortening was measured. Myocytes harboring the mutant transgenes were identified by co-transfection of adenoviral vectors expressing GFP as a marker.

## EXAMPLE 4

Construction of a PLB inhibitor-transport peptide complex

A PLB inhibitor molecule was made by indirectly attaching a transport peptide and a PLB protein to a polylysine backbone. Alternatively, the PLB molecule could also have been made as a single long peptide consisting of a transport sequence tandemly attached to the cargo peptide sequence. The transport peptide was composed of residues 43 to 58 of antennapedia (Seq. ID. No. 7), a Drosophila transcription factor protein. The cargo peptide was derived using the first 16 residues of PLB (Seq. ID. No. 8). It is important to note that this cargo sequence could also have been derived from any segment of wild-type PLB or mutant PLB.

The PLB inhibitor molecule was constructed by linking 4 transport peptides with 4 peptides matching the first 16 residues of PLB. The backbone linker was an 8-branch lysine, commonly used in multiple antigenic peptide (MAP) synthesis. The first 4 branches of the MAP resin were used to synthesize the antennapedia peptide. The next 4 branches were then deprotected and used as the starting point for the synthesis of the PLB cargo peptide. Thus, this particular PLB inhibitor that was used for initial characterization had 4 branches of the antennapedia peptide and 4 branches of the PLB cargo peptide. Alternatively, the PLB inhibitor could have been constructed as a single peptide with the cargo and transport peptides attached to each other by a single peptide bond, or as the cargo and transport peptides attached to each other by a disulfide bond. The PLB inhibitor molecule was translocated efficiently into isolated neonatal rat cardiomyocytes and showed a resulting enhanced contractility of the cell, the results of which can be seen in Figures 5a and b. Myocytes that overexpressed the V49A PLB point mutation showed increased contractility, while myocytes which overexpressed the wild-type PLB exhibited decreased contractility when compared to non-infected myocytes.

**EXAMPLE 5**

Penetratin peptides TAT and ANT

Cell level studies were done to evaluate the ability of two penetratin -based peptides, two mutant PLB-penetratin peptides, and two multiple antigen peptides (MAP) to strengthen the contraction cycle of isolated mouse cardiomyocytes. The two penetratin-based peptides include PLB-ANT (Seq. ID. No. 10) and TAT-PLB (Seq. ID. No. 11) each of which have a 20 residue portion of the PLB sequence attached to either the 5' end of ANT (Seq. ID. No. 14) or the 3' end of TAT (Seq. ID. No.15). The two mutant PLB peptides, mutant PLB-ANT (Seq. ID. No. 12) and TAT-mutant PLB (Seq. ID No. 13), display a S16E mutation of the 20 residue PLB sequence. The multiple antigen peptides include MAP with 8 penetratin (ANT) domains and MAP with 4 penetratin domains and 4 PLB domains .

Each of the penetratin-PLB peptides were evaluated to measure their ability to strengthen the contraction cycle of isolated mouse cardiomyocytes, the idea being that the penetratin-PLB peptide would act as a dominant negative inhibitor of the PLB-SERCA2a interaction. The results of the TAT-PLB peptide on the isolated cardiomyocytes is shown in Table 3. For this data, tests were repeated on several sets of cardiomyocytes to determine relative change in length through the contraction cycle with the TAT-PLB peptide (samples 1-7) and without the peptide (controls 1-8). Each of the samples had an added concentration of 10 $\mu$M of the TAT-PLB peptide while the controls had no added peptide.

Table 3

| | Maximum Contract. | Minimum Contract. | % Contract. | Δ Length/ msec. | r2 (fit) | Contract. Slope | Relax. Slope |
|---|---|---|---|---|---|---|---|
| control 1 | 100.63 | 92.978 | 7.604 | -34.503 | 0.9696 | 29.935 | 0.9955 |
| control 2 | 91.146 | 83.301 | 8.607 | -38.76 | 0.9943 | 28.736 | 0.9349 |
| control 3 | 115.45 | 100.05 | 13.339 | -82.875 | 0.9768 | 89.054 | 0.9894 |
| control 4 | 105.00 | 102.04 | 2.819 | -19.196 | 0.9842 | 10.497 | 0.9944 |
| control 5 | 83.747 | 79.637 | 4.908 | -21.695 | 0.9971 | 12.184 | 0.9742 |
| control 6 | 145.96 | 136.85 | 6.241 | -50.185 | 0.9721 | 27.566 | 0.9912 |
| control 7 | 154.56 | 142.16 | 8.023 | -76.607 | 0.9933 | 73.70 | 0.9928 |
| control 8 | 115.59 | 102.68 | 11.169 | -59.643 | 0.9765 | 63.304 | 0.9789 |
| **Mean** | **114.010** | **104.962** | **7.839** | **-47.933** | **0.9830** | **41.872** | **0.9814** |
| sample 1 | 112.57 | 101.17 | 10.127 | -65.554 | 0.9865 | 59.875 | 0.9925 |
| sample2 | 109.68 | 102.00 | 7.002 | -30.267 | 0.9609 | 37.157 | 0.9790 |
| sample3 | 133.82 | 116.32 | 13.077 | -79.242 | 0.9964 | 134.46 | 0.9878 |
| sample 4 | 81.61 | 67.871 | 16.835 | -58.093 | 0.9961 | 65.017 | 0.9697 |
| sample 5 | 74.423 | 64.629 | 13.160 | -54.353 | 0.9539 | 47.775 | 0.9933 |
| sample 6 | 126.89 | 108.38 | 14.587 | -98.054 | 0.9819 | 107.07 | 0.9939 |
| sample 7 | 133.61 | 128.21 | 4.042 | 36.071 | 0.9966 | 27.911 | 0.9959 |
| **Mean** | **110.373** | **98.363** | **11.261** | **-60.233** | **0.9818** | **68.466** | **0.9874** |

Measurements were taken every 20 milliseconds, and the unit of length was arbitrary, but was generally on the order of one complete cell length. Percent contraction was calculated as (maximum length minus minimum length) divided by maximum length. A plot of time versus length generated a U-shaped curve from which the most linear segments were selected, with the left side of the "U" representing contraction and the right side representing relaxation. The r2 column shows how well the data fit the curve, where 1.0 represents a perfect fit.

While there appeared to be a trend towards a larger, faster contraction in the myocyte, T-test analysis not identify any statistical difference due to the high variability of the data.

**EXAMPLE 6**

Hexahistidine tagged penetratin

A number of DNA constructs were generated for producing hexahistidine (H6) tagged penetratin and penetratin-PLB recombinant proteins in bacteria. Using a commercially available expression vector, pRSET (Invitrogen), recombinant protein H6-ANT (Seq. ID. No16) was generated. While this recombinant protein has no PLB attached, it was engineered to have epitope tags which was used to detect the protein as it entered the heart. A variation of H6-ANT was also expresses containing the PLB sequence, H6-wtPLB-ANT (Seq. ID. No. 17), in addition to an H6-PLB(S16E mutant)-ANT protein and an H6-PLB (V49A mutant)-ANT protein (Seq. ID. Nos. 18 and 19 respectively). H6-beta-galactosidase-ANT, H6-TAT, and H6-beta-galactosidase-TAT were also expressed at lower levels (seq. not listed). A non-functional ANT-penetratin with two mutations at residues 68 and 67, where Trp was mutated to Phe were made as negative control for the other three penetratin-PLB proteins.

To measure the effect these recombinant penetratin-based proteins have on cardiac contraction, one mouse was injected intraperitoneally with 2 mg of the H6-ANT peptide. A second mouse was injected intraperitoneally with 2 mg of the H6-ANT mutant protein. After a 3 hour incubation period, the mice were sacrificed and the hearts removed for analysis. The blood in the heart was removed by forcing fluid backwards through the aortic arch. Each heart was dissected into atrial tissue, left ventricular tissue, and right ventricular tissue. All the tissue was washed extensively in a physiologically balanced PBS solution and flash frozen in liquid nitrogen. The tissue were then lysed in 8 M Urea, 2% triton-X100, for 10 minutes and equal amounts of the supernatants were electrophoresed on 15% PAGE. The bands were transferred to a PVDF membrane. The membranes were labeled with anti-His antibody in order to identify if the lysate contained the epitope tagged protein.

The invention disclosed herein provides several methods for the treatment of heart failure through the inhibition or alteration of the interaction between phospholamban and sarcoplasmic reticulum $Ca^{2+}$ - ATPase within the cardio-myocytes. Although the invention has been described with reference to the examples provided above, it should be understood that various modifications can be made without departing from the spirit of the invention. Accordingly, the invention is limited only by the following claims.

**Claims**

1. A method for treatment of heart failure comprising inducing phospholamban deficiency.

2. The method for treatment of heart failure of claim 1, wherein an exogenous phospholamban protein induces phospholamban deficiency.

3. The method for treatment of heart failure of claim 2, wherein the exogenous phospholamban protein is selected from the group consisting of mutations of PLB, sense PLB, antisense PLB, truncated PLB, native PLB, and antibody against PLB.

4. The method for treatment of heart failure of claim 3, wherein the mutations of PLB comprise point mutations of PLB.

5. The method for treatment of heart failure of claim 3, wherein the antibody against PLB comprises contractilin.

6. A peptide based therapeutic agent for inhibiting phospholamban activity consisting of a first peptide and a second peptide as a complex, wherein the first peptide comprises a transport peptide and the second peptide comprises a cargo peptide.

7. The peptide based therapeutic agent of claim 6, wherein the transport peptide is selected from the group consisting of penetratin, adenovirus , bacterial and lipid vesicle based transport peptide.

8. The peptide based therapeutic agent of claim 6, wherein the cargo peptide is selected from the group consisting of mutations of PLB, sense PLB, antisense PLB, truncated PLB, and native PLB protein.

9. The peptide based therapeutic of claim 6, wherein the first peptide transports the second peptide across a cell membrane.

10. The peptide based therapeutic of claim 6, wherein the first peptide and the second peptide are linked by a covalent linkage.

11. The peptide based therapeutic of claim 10, wherein the covalent linkage consists of a branched polylysine backbone, a single peptide bond, or a disulfide bond.

12. A method for treatment of heart failure comprising enhancement of cardiac contractility by inhibition of PLB-SERCA2a interaction.

13. The method of claim 12, wherein the cardiac contractility is enhanced by inhibiting effect of PLB on sarcoplasmic reticulum $Ca^{2+}$ ATPase.

14. The method of claim 12, wherein an exogenous phospholamban protein is used to inhibit phospholamban deficiency.

15. The method of claim 14, wherein the exogenous phospholamban protein is selected from the group consisting of mutations of PLB, sense PLB, antisense PLB, truncated PLB, native PLB, and antibody against PLB.

16. The method of claim 15, wherein the mutations of PLB comprise point mutations of PLB.

17. The method for treatment of heart failure of claim 15, wherein the antibody against PLB comprises contractilin.

**Fig. 1**

## Fig. 2a

## Fig. 2b

## Fig. 2c

## Fig. 2d

## Fig. 2

**Fig. 2e**

**Fig. 2f**

**Fig. 2g**

**Fig. 2h**

Dobutamine (μg/kg/min)

**Fig. 2**

Fig. 3a

Fig. 3b

Fig. 3c

Fig. 3d

Fig. 3

Fig. 3 e

Fig. 4a

b

Fig. 4b

**Fig. 5**

**Fig. 5a**

Myocyte Length Change (%) vs Time (sec): control, wildtype PLB, V49A PLB

**Fig. 5b**

|  | control n=7 | wildtype PLB n=3 | V49A mutant PLB n=4 |
|---|---|---|---|
| Shortening (%) | 7.20 ± 0.60 | 3.50 ± 0.24 * | 11.45 ± 0.88 ** |
| -dL/dt (mm) | 88.4 ± 10.3 | 48.4 ± 5.9 * | 150.3 ± 9.6 **† |
| +dL/dt (mm) | 56.6 ± 6.5 | 28.1 ± 6.4 † | 116.5 ± 13.5 **† |
| Cell length (μm) | 104.5 ± 3.1 | 109.2 ± 3.9 | 108.7 ± 3.3 |

EP 1 728 516 A1

**Fig. 6a**

**Fig. 6b**

**Fig. 6c**

**Fig. 6**

**Fig. 7**

**Fig. 8**

**European Patent Office**

# EUROPEAN SEARCH REPORT

Application Number

EP 06 11 6525

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KOSS K L ET AL: "PHOSPHOLAMBAN: A PROMINENT REGULATOR OF MYOCARDIAL CONTRACTILITY" CIRCULATION RESEARCH, vol. 79, no. 6, 1 December 1996 (1996-12-01), pages 1059-1063, XP002053754 ISSN: 0009-7330 * the whole document * | 1-5, 12-17 | INV. A61K38/17 A61K47/48 C07K17/00 A61P9/04 |
| X | DILLMANN W H: "Influences of increased expression of the Ca2+ ATPase of the sarcoplasmic reticulum by a transgenic approach on cardiac contractility." ANNALS OF THE NEW YORK ACADEMY OF SCIENCES, 853 43-8., 16 September 1998 (1998-09-16), XP000914521 * page 46, line 47 - page 47, line 16 * | 1-5, 12-17 | |
| X | HE HUAPING ET AL: "Influence of an antisense phospholamban transcribed by an adenoviral vector on Ca-2+ ATPase in cardiac myocytes." JOURNAL OF MOLECULAR AND CELLULAR CARDIOLOGY, vol. 29, no. 6, 1997, page A181, XP000907558 ISSN: 0022-2828 * the whole document * | 1-5, 12-17 | TECHNICAL FIELDS SEARCHED (IPC) A61K C07K |
| X | WO 97/37224 A (MEDICAL RES COUNCIL ;BARFORD DAVID (GB); COHEN PHILIP (GB); COHEN) 9 October 1997 (1997-10-09) * page 32, line 1 - line 7 * | 1,12,13 | |

-/--

The present search report has been drawn up for all claims

| Place of search | Date of completion of the search | Examiner |
|---|---|---|
| Munich | 12 September 2006 | Didelon, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

European Patent
Office

**EUROPEAN SEARCH REPORT**

Application Number

EP 06 11 6525

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FR 2 753 722 A (SMITHKLINE BEECHAM LAB) 27 March 1998 (1998-03-27) * the whole document * | 1,12,13 | |
| D,A | TOYOFUKU T ET AL: "Amino acids Glu2 to Ile18 in the cytoplasmic domain of phospholamban are essential for functional association with the Ca2+-ATPase of sarcoplasmic reticulum" THE JOURNAL OF BIOLOGICAL CHEMISTRY, vol. 269, no. 4, 28 January 1994 (1994-01-28), pages 3088-3094, XP002139384 * the whole document * | 1-5, 12-17 | |
| A | US 5 652 122 A (PABO CARL ET AL) 29 July 1997 (1997-07-29) * the whole document * | 6-11 | |
| P,X | MINAMISAWA SUSUMU ET AL: "Chronic phospholamban-sarcoplasmic reticulum calcium ATPase interaction is the critical calcium cycling defect in dilated cardiomyopathy." CELL, vol. 99, no. 3, 29 October 1999 (1999-10-29), pages 313-322, XP002139385 ISSN: 0092-8674 * the whole document * | 1-5, 12-17 | TECHNICAL FIELDS SEARCHED (IPC) |

-/--

The present search report has been drawn up for all claims

| Place of search | Date of completion of the search | Examiner |
|---|---|---|
| Munich | 12 September 2006 | Didelon, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 06 11 6525

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,X | HE HUAPING ET AL: "Effects of mutant and antisense RNA of phospholamban on SR Ca2+-ATPase activity and cardiac myocyte contractility." CIRCULATION, vol. 100, no. 9, 31 August 1999 (1999-08-31), pages 974-980, XP000907563 ISSN: 0009-7322 * the whole document * ----- | 1-17 | |
| P,X | WO 99/30696 A (HAIKALA HEIMO ;OVASKA MARTTI (FI); KAHEINEN PETRI (FI); KAIVOLA JU) 24 June 1999 (1999-06-24) * the whole document * ----- | 1,12,13 | |

TECHNICAL FIELDS SEARCHED (IPC)

The present search report has been drawn up for all claims

| Place of search | Date of completion of the search | Examiner |
|---|---|---|
| Munich | 12 September 2006 | Didelon, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 1 728 516 A1

**ANNEX TO THE EUROPEAN SEARCH REPORT
ON EUROPEAN PATENT APPLICATION NO.**

EP 06 11 6525

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2006

| Patent document cited in search report | | Publication date | Patent family member(s) | | Publication date |
|---|---|---|---|---|---|
| WO 9737224 | A | 09-10-1997 | AU | 722840 B2 | 10-08-2000 |
| | | | AU | 2172597 A | 22-10-1997 |
| | | | CA | 2250826 A1 | 09-10-1997 |
| | | | EP | 0891551 A1 | 20-01-1999 |
| | | | JP | 2000508761 T | 11-07-2000 |
| | | | US | 6884600 B1 | 26-04-2005 |
| FR 2753722 | A | 27-03-1998 | WO | 9813518 A1 | 02-04-1998 |
| US 5652122 | A | 29-07-1997 | US | 5674980 A | 07-10-1997 |
| | | | US | 5670617 A | 23-09-1997 |
| | | | US | 5747641 A | 05-05-1998 |
| WO 9930696 | A | 24-06-1999 | AT | 224187 T | 15-10-2002 |
| | | | AU | 1565599 A | 05-07-1999 |
| | | | BR | 9813549 A | 10-10-2000 |
| | | | CA | 2311932 A1 | 24-06-1999 |
| | | | DE | 69808131 D1 | 24-10-2002 |
| | | | DE | 69808131 T2 | 05-06-2003 |
| | | | DK | 1039884 T3 | 09-12-2002 |
| | | | EP | 1039884 A1 | 04-10-2000 |
| | | | ES | 2184341 T3 | 01-04-2003 |
| | | | JP | 2002508314 T | 19-03-2002 |
| | | | PT | 1039884 T | 31-01-2003 |
| | | | US | 5968959 A | 19-10-1999 |
| | | | ZA | 9811180 A | 09-06-1999 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10671898 P
- US 14588399 P

**Non-patent literature cited in the description**

- **MERCADIER et al.** *J. Clin. Invest.,* 1990, vol. 85, 305-309
- **ARAI et al.** *Circ. Res.,* 1993, vol. 72, 463-469
- **BASTIE et al.** *Circ. Res.,* 1990, vol. 66, 554-564
- **FELDMAN et al.** *Circ. Res.,* 1993, vol. 73, 184-192
- **TADA et al.** *J. Bio.l Chem.,* 1974, vol. 249, 6174-6180
- **TOYOFUKU et al.** *J. Biol. Chem.,* 1994, vol. 269, 3088-3094
- **ARBER et al.** *Cell,* 1997, vol. 88, 393-403
- **SCHWARZE et al.** *Science,* 1999, vol. 285, 1569-1572
- **ROCKMAN et al.** *Proc. Natl. Acad. Sci. USA,* 1998, vol. 95, 7000-7005